# EUROPEAN PATENT APPLICATION

(11) **EP 4 636 661 A1**
(43) Date of publication of application: **22.10.2025**
(21) Application number: 24170229.9
(22) Date of filing: 15.04.2024
(51) Int. Cl.: G06N 20/00, G06F 16/55, G16H 30/40

(54) **TRAINING-DEVICE AND METHOD FOR TRAINING A MACHINE LEARNING MODEL**

(71) Applicant: Siemens Healthineers AG, 91301 Forchheim (DE)
(72) Inventor: Biniazan, Ramyar, 90402 Nürnberg (DE); Fieselmann, Andreas, 91052 Erlangen (DE); Fok, Wai Yan Ryana, 85748 Garching b. München (DE); Hümmer, Christian, 96215 Lichtenfels (DE)
(74) Representative: Siemens Healthineers Patent Attorneys

(57) **Abstract**

The invention pertains to a training-device (1) for training a machine learning model (M), the training-device (1) comprising a training-unit (2), a testing-unit (3) and a data-generator (4), wherein
- the data-generator (4) is designed to generate synthetic training-images (I_{S}) from a first set of parameter-values (P1) and synthetic testing-images (T_{S}) from a second set of parameter-values (P2), wherein all parameter-values (P1, P2) are values of a common predefined parameter-set, and each image is linked to a ground truth,
- the training-unit (2) is designed to train a machine learning model (M) with training-images (I) in a supervised manner,
- the testing-unit (3) is designed to
a) test a trained model (M) based on testing-images (T),
b) evaluate individual test-qualities of the test for single testing-images (T_{S}), and
c) determine parameter value-ranges for groups of testing-images (T) representing ranges with a relatively poor image-density, where test-qualities are below a predefined threshold value, and/or the relative number of training-images (I) compared to other parameter value-ranges is below a predefined threshold,

wherein the training-device (1) is further designed to feed the parameter value-ranges (V) back to the data-generator (4), generate additional synthetic training-images (I_{S}) based on the determined parameter value-ranges (V), wherein each synthetic training-images (I_{S}) is linked to a ground truth, and further train the model (M) with the additional synthetic training-images (I_{S}). Furthermore, the invention describes a respective method.

## Description

The invention describes a training-device and a method for training a machine learning model.

Deep Learning has revolutionized medical software applications by improving diagnostic accuracy, enabling workflow automation, and enhancing patient care. However, the effectiveness of deep learning models in healthcare relies on representative data, particularly in recognizing rare medical conditions. An under-representation of training data can lead to poor model performance and impede their full potential in addressing critical medical challenges. In general, the problem lies in an under-representation in training data and/or an under-representation in testing data.

When deep learning models are trained on datasets that under-represent rare conditions ("under-representation in training data"), they may not learn to recognize these conditions effectively. The model tends to bias toward the majority class, leading to poor performance when it encounters rare conditions in real-world scenarios.

On the other hand, by not providing enough rare cases in testing dataset ("under-representation in testing data"), the performance of trained models on rare cases is unknown and therefore not reliable.

Previous studies also explored the use synthetic data to compensate underrepresented cases in either training and testing or both.

Concerning training, the study by Sukesh et al. 2022 ("Training deep learning models for 2D spine x-rays using synthetic images and annotations created from 3D CT volumes", Bildverar-beitung für die Medizin 2022: Proceedings, German Workshop on Medical Image Computing, Heidelberg, (pp. 63-68), June 26-28, 2022) added synthetic spine X-ray data from CT volumes to real X-ray data to improve the network performance in vertebrae detection. The study from Gao et al. 2023 ("Synthetic data accelerates the development of generalizable learning-based algorithms for X-ray image analysis", Nature Machine Intelligence, 5(3), 294-308, 2023) used synthetic X-ray data from realistic simulation and strong domain randomization of CT volumes to train models for clinical tasks such as surgical tool and landmark detection, hip and lung lesion segmentation. Both studies used synthetic X-ray in training and real X-ray for testing.

Concerning testing, the study by Fok et al. 2023 ("Learning Patient Rotation Using Synthetic X-ray Images from 3D CT Volumes", Medical Imaging with Deep Learning, short paper track", 2023) used synthetic X-ray data from CT volumes to train and test the model for learning internal patient rotation. The estimation of patient rotation angle is less accurate in large angles, but no feedback is yet provided to generate more synthetic X-ray with large angles for further training.

However, the current challenge is whether the synthetic data could cover all rare conditions and the combinations of them, in terms of patient positioning, pathologies, image quality, etc.

The current methods only showed the direct generation of synthetic X-ray data from CT data for training without evaluating the synthetic data in testing nor giving feedback to the synthetic data generator.

Therefore, our invention aims to improve the synthetic data generation based on testing performance by generating new combinations of underrepresented distribution in different conditions, so to ensure the trained network (model) is robust to different multivariate distribution.

It is the object of the present invention to improve the known systems and methods and provide a training-device and a method for training a machine learning model for overcoming the above described problems.

This object is achieved by a training-device according to claim 1 and a method according to claim 7.

A training-device according to the invention serves for training a machine learning model. It comprises a training-unit, a testing-unit and a data-generator.

The data-generator is designed to generate synthetic training-images from a first set of parameter-values and synthetic testing-images from a second set of parameter-values, wherein all parameter-values are values of a common predefined parameter-set, and each image is linked to a ground truth.

The training-unit is designed to train a machine learning model with training-images in a supervised manner.

The testing-unit is designed to
a) test a trained model based on testing-images,
b) evaluate individual test-qualities of the test for single testing-images, and
c) determine parameter value-ranges for groups of testing-images representing ranges with a relatively poor image-density, where test-qualities are below a predefined threshold value, and/or the relative number of training-images compared to other parameter value-ranges is below a predefined threshold.

The training-device is further designed to feed the parameter value-ranges back to the data-generator, generate additional synthetic training-images based on the determined parameter value-ranges, wherein each synthetic training-image is linked to a ground truth, and further train the model with the additional synthetic training-images.

Training-devices comprising a training-unit and a testing-unit are well known in the art. They could train a machine learning model with training-images and use testing-images for testing the state and the capabilities of the trained model. As said above, Training could be performed with real training-images and also with synthetic training-images.

Machine learning models are well known in the art. For segmenting-images, especially convolutional networks are advantageous for the invention, since they could be used for effectively segment images.

Training-units designed to train a machine learning model with training-images in a supervised manner are well known in the art. They can be fed with training-images comprising or linked to a ground truth and train a machine learning model. The training-images may comprise the ground truth, e.g. labels labeling certain areas of the training-images or be linked to a ground truth, e.g. the training-unit knows that the next images all show certain objects.

Testing-units designed to test a trained model based on testing-images and evaluate individual test-qualities of the test for single testing-images are also well known.

The training device according to the invention is special, because it also comprises a data-generator and is designed to feed back parameter value-ranges of very special results from the testing step in order to selectively generate special training-images.

Also, data-generators are known in the art, that are able to generate synthetic images (training-images as well as testing-images) with or without a ground truth.

To begin with, the testing-unit has the special ability to determine parameter value-ranges for groups of testing-images representing ranges with a relatively poor image-density. In these ranges, test-qualities are below a predefined threshold value, and/or the relative number of training-images compared to other parameter value-ranges is below a predefined threshold.

The parameters of the images should generally stay the same, e.g. parameters concerning-image acquisition (e.g. parameters of an X-ray beam), the position of the recorded object, the angle of view, and maybe environmental conditions. However, the values of the parameters vary over the training-images. Now, there may be many images taken with "normal" parameter values and only a few images taken with "exotic" parameter values.

Typically, the testing with testing-images acquired with "exotic" parameter values will result in poor testing results, since also the training-images with "exotic" parameter values were not sufficient for training due to the low number. Thus, the test-qualities of such images are generally below a predefined threshold value. In addition or alternatively, the images could also be simply counted. In the case there are only a few images in a certain value-range (while there are many images in other value-ranges comparable in range-size), it could be decided that the relative number of training-images compared to other parameter value-ranges is below a predefined threshold and that there is a poor image density. However, since it is not always known, whether a certain value-range is of special interest, testing results are preferred for determining whether there is a poor image density or not.

Thus, the testing-unit is designed to output value-ranges for a certain number of parameters where a poor image density has been detected. This value-range may concern only one single parameter, e.g. a certain angle of view or a certain beam intensity, but may also concern two or more parameters. e.g. a certain angle of view at a certain beam intensity. However, this issue may be getting complicated with a great number of parameters.

Concerning the value-ranges, the training-device is designed to feed the parameter value-ranges back to the data-generator.

The data-generator of the training device according to the invention is designed to generate synthetic training-images (with a ground truth or linked to a ground truth) from a given set of parameter-values. Since the parameter-values used for training-images and testing-images may differ, it is said that a first set of parameter-values is used for generating synthetic training-images and a second set of parameter-values is used for generating synthetic testing-images. In general, the first set of parameter-values Θt (training) and the second set of parameter-values Θv (testing or "validation") are preferably different because only Θt is updated and Θv is left unchanged (as it is advantageous for testing consistency when the validation images are not changing). It should be noted that the result on the validation dataset can be evaluated and if seen that cases with certain parameter values tend to fail, those parameter values could be overrepresented in Θt (e.g. in terms of numbers of samples to be created).

When there is a number of value-ranges fed back by the training device, the data-generator will generate synthetic images (that are then used as training-images, but may also be used as testing-images) based on the determined parameter value-ranges. Again, each synthetic training-image is linked to a ground truth for supervised training. It is preferred that the images are generated by clinging to the given parameter value-ranges for certain parameters, and to vary parameter values for other parameters to get a broader bandwidth of possible cases. Thus, in the case there is a value-range given for the angle of view, the generator varies in this range only for the angle of view, but varies broadly over beam intensity not restricted. There could easily be defined change-ranges in which certain parameters can change in one of said value-ranges.

With these additional synthetic training-images the model is further trained. Since they are pertaining to certain parts of the parameter values where there have been only a few images, the model could purposefully be trained for a certain "rare condition".

A method according to the invention serves for training a machine learning model. The method is performed with a training-device according to the invention and comprises the following steps:
a) providing training-images and testing-images,
b) training of the model with the training-images,
c) testing the trained model based on the testing-images, wherein individual test-qualities are evaluated for single testing-images, and parameter value-ranges are determined for groups of testing-images, representing ranges with a relatively poor image-density, where test-qualities are below a predefined threshold value, and/or the relative number of training-images compared to other parameter value-ranges is below a predefined threshold,
d) generating synthetic training-images and preferably also synthetic testing-images with the data-generator based on the evaluated set of parameter value-ranges,
e) repeating at least steps b) and c) with the generated synthetic training-images and preferably also with generated synthetic testing-images.

The functions of the components of the training-device have been explained above.

First, there are provided training-images and testing-images (with or combined with a ground truth). These images could be real images or synthetic images or both. Since an important application is medicine especially X-ray imaging, the images could be medical images, especially X-ray images.

Then, the machine learning model is trained with the training-images. Such training is well known in the art.

After that, the trained model is tested based on the testing-images. In the course of this testing, individual test-qualities are evaluated for single testing-images. These test-qualities are important for evaluating the trained model, but could also be used for the training of "rare conditions".

Also in the course of the testing, parameter value-ranges are determined for groups of testing-images, representing ranges with a relatively poor image-density. Such ranges with a relatively poor image-density are ranges of parameter values where test-qualities are below a predefined threshold value and/or the relative number of training-images compared to other parameter value-ranges is below a predefined threshold. Thus, poor image density may be found by poor testing results or maybe also by counting-images in certain value-bins. Generally, it could be said, where there are only a few images, training of the model will be not very effective. The regions where there are only a few images could be detected by counting-images in value-range-bins or by testing the model on testing-images.

In the case, value-ranges are found with a relatively poor image-density, the training could be biased for these ranges by providing more images of these value-ranges and training the model with these images.

Thus, in the next step, synthetic training-images are generated with the image generator based on the evaluated set of parameter value-ranges. Also synthetic testing-images could be generated with the data-generator in order to specifically test the "rare condition", however, this is not absolutely necessary.

Now, at least steps b) and c) of the method (training and testing) are repeated with the new synthetic training-images. The testing may be done with new synthetic testing-images or with the old images.

For better results, more iterations could be made by determining other value-ranges with "rare condition" and producing training-images (and possibly also testing-images) for different value-ranges. Every new cycle will be the proceeding of steps d), e), b) and c).

Some units or modules of the invention mentioned above can be completely or partially realized as software modules running on a processor of a computing system. A realization largely in the form of software modules can have the advantage that applications already installed on an existing computing system can be updated, with relatively little effort, to install and run these units of the present application. The object of the invention is also achieved by a computer program product with a computer program that is directly loadable into the memory of a computing system, and which comprises program units to perform the steps of the methods, at least those steps that could be executed by a computer, when the program is executed by the computing system. In addition to the computer program, such a computer program product can also comprise further parts such as documentation and/or additional components, also hardware components such as a hardware key (dongle etc.) to facilitate access to the software.

A computer readable medium such as a memory stick, a hard-disk or other transportable or permanently-installed carrier can serve to transport and/or to store the executable parts of the computer program product so that these can be read from a processor unit of a computing system. A processor unit can comprise one or more microprocessors or their equivalents.

Particularly advantageous embodiments and features of the invention are given by the dependent claims, as revealed in the following description. Features of different claim categories may be combined as appropriate to give further embodiments not described herein.

It is preferred that the data-generator is designed to generate synthetic medical images, preferably synthetic X-ray images in form of projection images and/or tomographic images. It is particularly preferred that a set of parameter-values comprises values of one or more parameters of the group X-ray tube viewing angle, patient positioning, collimation, image quality (especially noise or sharpness), X-ray post-processing parameters (e.g. flavors), anatomical variations, anatomical pathologies and foreign objects (e.g. implants, lines or tubes).

In a preferred case, there are used three dimensional images, especially CT-images, in order to generate synthetic X-ray projection images. A projection vector could be defined for a 3D-image defining the angle of view and the 3D-image could be projected on a plane having the projection vector as normal plane-vector. Thus, preferably, the data-generator is designed to generate synthetic X-ray projection images generated by forward projecting 3D computertomographic images on a 2D-plane.

It is further preferred that the data-generator is designed to emulate a virtual detector acquiring the projected image and preferably also to process the resulting-image with a number of X-ray image processing algorithms. Such algorithms are known in the art and could be used to slightly alter images concerning special parameters.

A preferred embodiment of the training-device comprises a data-interface designed to receive and/or fetch real training-images. and wherein the training-device is designed to use the real images as training-images for training the model and/or as testing-images for testing the trained model. A mixture of real and synthetic training-images is advantageous, since the synthetic images are numerous and the real images show real content.

A preferred embodiment of the training-device is designed to evaluate recording-parameter-values of the real images. These real images could then be used as testing-images. In addition, the training-device is preferably designed to count the real images for predefined value-ranges of the recording-parameter-values, determine a number of value-ranges with the fewest number of images and feed these value-ranges back to the data-generator in order to create additional synthetic training-images. Thus, "rare conditions" of real images could be determined and additional synthetic training-images could be provided to train the model for these rare conditions.

As said above, the testing is a very good indicator of rare conditions. Thus, the testing-unit is preferably designed for:
- selecting testing-images with test qualities below a predefined quality-threshold,
- evaluating patterns in the second sets of parameter-values of these testing-images (in order to estimate value-ranges of "rare conditions"),
- determining parameter value-ranges from these patterns,
- forming a first set of parameter-values based on the determined value-ranges.

Preferably, the training-device comprises a pre-augmentation-unit designed to evaluate the (synthetic, but possibly also real) training-images created by the data-generator for their value for training and delete images not usable for training. Preferably, the pre-augmentation-unit is designed to determine a numerical value, compare this value with a numerical range and delete an image or mark an image to be deleted in the case the value lies out of the range.

Regarding a preferred method, for step a) (providing training-images and testing-images), at least a part of the training-images and/or the testing-images are synthetic images generated by the data-generator.

Concerning a preferred method, the image-density of parameter value-ranges are evaluated by evaluating the performance on the testing of testing-images. When the trained model has poor performance on specific cases, respective parameter value-ranges are determined. In an easy example, the parameter values of said specific cases are examined and when there are certain groups of specific cases with similar parameter values, these parameter values could be taken as value-range for generating new training-images.

Preferably, the image-density of parameter value-ranges are evaluated by counting the images in each value-range and normalizing the counted values (in order to get value-ranges with relatively few images compared to other value-ranges). It is preferred that the image-density of parameter value-ranges are evaluated by counting the images with a specific test result (e.g. fail/pass) in each range and normalizing the counted values.

Preferably, parameter value-ranges are defined by prior knowledge about a relatively poor image-density in these value-ranges and synthetic training-images are generated by the synthetic data generator, and preferably also synthetic testing-images for testing.

Preferably, multiple training-iterations are performed with synthetic training-images, and especially also with synthetic testing-images, based on certain sets of parameter-values. It is particularly preferred that during each training iteration sets of parameter-values are evaluated from the testing-images and if testing-images of a set of parameter-values perform poorly, this set of parameter-values is prioritized as new first set of parameter-values in the next iteration, prompting the generator to produce more data with those first set of parameter-values.

Preferably, the training is based on supervised learning, while each training-image and testing-image is connected with or comprises an individual ground truth, preferably wherein the training is performed in order to segment the images and/or classify elements of the images and/or detect objects in the images. Preferably, the model is trained for bone segmentation in an image.

The methods may also include elements of "cloud computing". In the technical field of "cloud computing", an IT infrastruc-ture is provided over a data-network, storage space or processing power and/or application software. The communication between the user and the "cloud" is achieved by means of data interfaces and/or data transmission protocols. In the context of "cloud computing", in a preferred embodiment of the methods according to the invention, provision of data via a data channel (for example a data-network) to a "cloud" takes place. This "cloud" includes a (remote) computing system, e.g. a computer cluster that typically does not include the user's local machine. It is particularly preferred that the cloud service provides as well computing power as application software.

Thus, the invention aims to improve the synthetic data generation based on testing performance by generating new combinations of underrepresented distribution in different conditions, so to ensure trained network is robust to different multivariate distribution. The advantages of the invention are the feedback loop that is used to update synthesis parameters in the data generator. Thus the data generator is by design creating samples with high relevance for the training. On the other hand, the workflow may include separate testing on real and synthetic data enabling testing on rare cases (mostly synthetic data) and also common cases (mostly real data). Applying this invention can mitigate underrepresented case in training and testing data.

Other objects and features of the present invention will become apparent from the following detailed descriptions considered in conjunction with the accompanying drawings. It is to be understood, however, that the drawings are designed solely for the purposes of illustration and not as a definition of the limits of the invention.
Figure 1 shows a training-device according to the invention,
Figure 2 shows a block diagram of the method according to the invention.

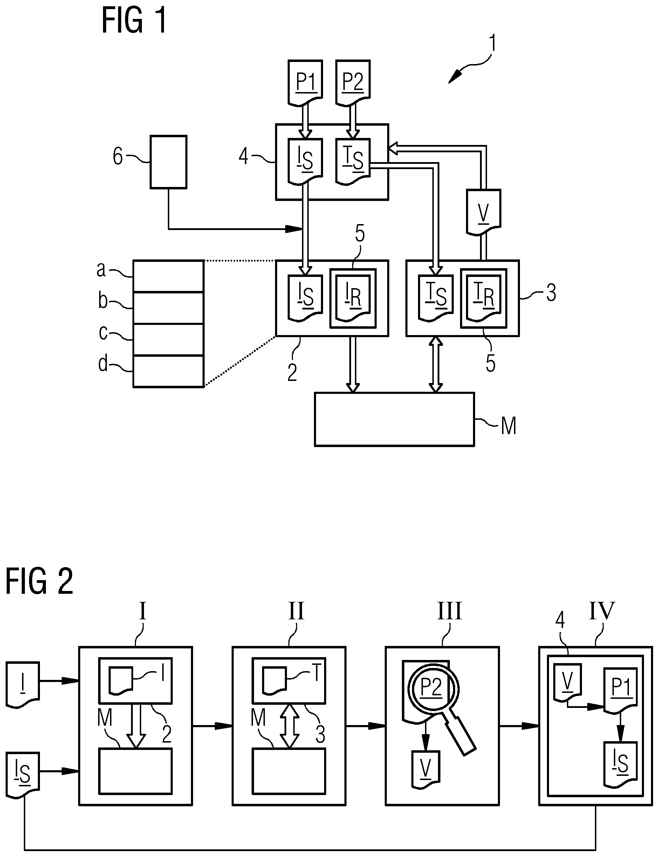

Figure 1 shows an example of a training-device 1 according to the invention for training a machine learning model M. The training-device 1 comprises a training-unit 2, a testing-unit 3 and a data-generator 4.

The training-unit 2 is designed to train a machine learning model M with training-images I in a supervised manner. In this example, it comprises an in-training augmentation unit a, a data preprocessing-unit b, an optimization unit c and a validation unit d. Such training-units are well known. The validation unit d is commonly used for checking if training works properly and to avoid overfitting. Also, the data in the validation unit d could be a subset of the training data. Thus the validation unit d is different from the testing-unit 3 of the invention.

In the shown example, the training-device 1 comprises a data-interface 5 designed to receive and/or fetch real training-images I_{R} and wherein the training-device 1 is designed to use the real images as training-images I_{R} for training the model M and/or as testing-images T_{R} for testing the trained model M.

The data-generator 4 is designed to generate synthetic training-images I_{S} from a first set of parameter-values P1 and synthetic testing-images T_{S} from a second set of parameter-values P2, wherein all parameter-values P1, P2 are values of a common predefined parameter-set, and each image I, T is linked to a ground truth.

The testing-unit 3 is designed to test a trained model M based on testing-images T, evaluate individual test-qualities of the test for single testing-images T, and determine parameter value-ranges V for groups of testing-images T representing ranges with a relatively poor image-density, where test-qualities are below a predefined threshold value, and/or the relative number of training-images I compared to other parameter value-ranges V is below a predefined threshold.

The training-device 1 is further designed to feed the parameter value-ranges V back to the data-generator 4, generate additional synthetic training-images I_{S} based on the determined parameter value-ranges V, wherein each synthetic training-image Is is linked to a ground truth, and further train the model M with the additional synthetic training-images I_{S}.

The images I, T may be X-ray images in form of projection images and/or tomographic images and the set of parameter-values P1, P2 could comprises values of one or more parameters of the group X-ray tube viewing angle, patient positioning, collimation, image quality, X-ray post-processing parameters, anatomical variations, anatomical pathologies and foreign objects. In that case, the data-generator 4 may be designed to generate synthetic X-ray projection images generated by forward projecting 3D computertomographic images on a 2D-plane, preferably wherein the data-generator 4 is designed to emulate a virtual detector acquiring the projected image and preferably also to process the resulting-image with a number of X-ray image processing algorithms.

As said above, a key component of the invention is the (synthetic) data generator 4. This component creates e.g. synthetic X-ray images Is, Ts given a set of synthesis parameters Θ (with defined parameter values). Examples for synthesis parameters can be: X-ray tube viewing angle, patient positioning, collimation and/or image quality (noise, sharpness) and/or X-ray post-processing parameters (flavors) and/or anatomical variations/pathologies, foreign objects (implants, lines and tubes). The task of this data generator 4 is to create different synthetic X-ray images from different synthesis parameters Θ. A practical implementation can be using CT volumes that are forward projected onto a virtual detector and the detector image is processed with x-ray image processing algorithms.

The data generator 4 may create images both for the training of the model M and for the testing of the model M. The parameter sets for creating the training data could be denoted as Θₜᵢ (i=1,... ,Nₜ; first set of parameter values P1) whereas Θᵥᵢ (i=1,... ,Nᵥ; second set of parameter values P2) denotes the parameter sets for the testing/validation data.

As shown above, a key element of the invention is to update the synthesis parameters (first set of parameter values P1) for the data generator 4 during training and/or after testing.

After Testing: Successful model training is determined by its performance on a testing dataset with real clinical cases. When the trained model M has poor performance on specific cases, this will guide the creation of a new set of first parameter values P1 for the data generator 4. This set is then used to represent specific data in both the training and validation datasets.

During Training: Synthetic data representing rare clinical images (based on rare pathologies, acquisition parameters, patient positioning, etc.) are incorporated into the training and validation datasets. Each training epoch evaluates parameter sets in the testing data. If a set performs poorly, it is prioritized in the next epoch, prompting the generator to produce more data with those parameters.

Figure 2 shows a block diagram of the method according to the invention for training a machine learning model M with a training-device 1 as e.g. shown in figure 1.

There are provided training-images I and testing-images T. The provision of training-images I is indicated at the left of the blocks. The training-images I and testing-images may be real images and/or synthetic images generated by the data-generator 4.

In step I, the model M is trained with the training-images I. The training is based on supervised learning, while each training-image and testing-image is connected with or comprises an individual ground truth, preferably wherein the training is performed in order to segment the images and/or classify elements of the images and/or detect objects in the images.

In step II, the trained model M is tested based on the testing-images T.

In step III, individual test-qualities are evaluated for single testing-images T, and parameter value-ranges V are determined for groups of testing-images T, representing value-ranges V with a relatively poor image-density, where test-qualities are below a predefined threshold value, and/or the relative number of training-images I compared to other parameter value-ranges V is below a predefined threshold.

The image-density of parameter value-ranges V could be evaluated by evaluating the performance of the testing with testing-images T, and when the trained model M has poor performance on specific cases, respective parameter value-ranges V are determined. However, they could also be evaluated by counting the images in each range and normalizing the counted values. Preferably, the image-density of parameter value-ranges V could be evaluated by counting the images with a specific test result in each range and normalizing the counted values.

In step IV, synthetic training-images I_{S} are generated with the data-generator 4 based on the evaluated set of parameter value-ranges V.

Then, the training and testing (steps I and II) are repeated with the generated synthetic training-images I_{S}. Steps III and IV could also be repeated (again followed by steps I and II) for another iteration. Multiple training-iterations could be performed with synthetic training-images I_{S}, based on certain sets of parameter-values P1, preferably wherein during each training iteration sets of parameter-values P1 are evaluated from the testing-images T and if testing-images T of a set of parameter-values P2 perform poorly, this set of parameter-values P2 is prioritized as new first set of parameter-values P1 in the next iteration, prompting the generator to produce more data with this first set of parameter-values P1.

For example, from the evaluation on another synthetic X-ray test data, it is found that the estimation of patient rotation angle is less accurate in large angles. However, there is not yet feedback to generate more synthetic X-ray with large angles for further training. The invention would first evaluate the trained model M in both real and synthetic test data. If the performance in synthetic data does not fulfill the criteria, the invention will evaluate the test data performance with respect to the parameter sets Θₜᵢ, to identify whether the 'rotation angle' is the parameter contributing to the performance. Then the invention will perform a search in this parameter space to identify which 'rotation angles' need to be fed to generator for generating new synthetic X-ray. Moreover, this 'rotation angle' parameter would be combined with other parameters, i.e. generate new parameter sets, such as pathological condition, noise, image quality when generating the new synthetic X-ray. For instance, by varying low to high noise, varying scatter effect, or by varying a pathological image impression.

Although the present invention has been disclosed in the form of preferred embodiments and variations thereon, it will be understood that numerous additional modifications and variations could be made thereto without departing from the scope of the invention. For the sake of clarity, it is to be understood that the use of "a" or "an" throughout this application does not exclude a plurality, and "comprising" does not exclude other steps or elements. The expression "a number of" means "at least one". The mention of a "unit" or a "device" does not preclude the use of more than one unit or device. Independent of the grammatical term usage, individuals with male, female or other gender identities are included within the term.

## Claims

1. A training-device (1) for training a machine learning model (M), the training-device (1) comprising a training-unit (2), a testing-unit (3) and a data-generator (4), wherein
- the data-generator (4) is designed to generate synthetic training-images (I_{S}) from a first set of parameter-values (P1) and synthetic testing-images (T_{S}) from a second set of parameter-values (P2), wherein all parameter-values (P1, P2) are values of a common predefined parameter-set, and each image (I, T) is linked to a ground truth,
- the training-unit (2) is designed to train a machine learning model (M) with training-images (I) in a supervised manner,
- the testing-unit (3) is designed to
a) test a trained model (M) based on testing-images (T),
b) evaluate individual test-qualities of the test for single testing-images (T), and
c) determine parameter value-ranges for groups of testing-images (T) representing ranges with a relatively poor image-density, where test-qualities are below a predefined threshold value, and/or the relative number of training-images (I) compared to other parameter value-ranges is below a predefined threshold,
wherein the training-device (1) is further designed to feed the parameter value-ranges (V) back to the data-generator (4), generate additional synthetic training-images (I_{S}) based on the determined parameter value-ranges (V), wherein each synthetic training-image (I_{S}) is linked to a ground truth, and further train the model (M) with the additional synthetic training-images (I_{S}).

2. The training-device (1) according to claim 1, wherein the data-generator (4) is designed to generate synthetic medical images, preferably synthetic X-ray images in form of projection images and/or tomographic images, preferably wherein a set of parameter-values (P1, P2) comprises values of one or more parameters of the group X-ray tube viewing angle, patient positioning, collimation, image quality, X-ray post-processing parameters, anatomical variations, anatomical pathologies and foreign objects.

3. The training-device (1) according to claim 2, wherein the data-generator (4) is designed to generate synthetic X-ray projection images generated by forward projecting 3D computertomographic images on a 2D-plane, preferably wherein the data-generator (4) is designed to emulate a virtual detector acquiring the projected image and preferably also to process the resulting-image with a number of X-ray image processing algorithms.

4. The training-device (1) according to one of the preceding claims, comprising a data-interface (5) designed to receive and/or fetch real training-images (I_{R}) and wherein the training-device (1) is designed to use the real images as training-images (I_{R}) for training the model (M) and/or as testing-images (T_{R}) for testing the trained model (M).

5. The training-device (1) according to claim 4, designed to evaluate recording-parameter-values of the real images count the real images for predefined value-ranges (V) of the recording-parameter-values, determine a number of value-ranges (V) with the fewest number of images and feed these value-ranges (V) back to the data-generator (4) in order to create additional synthetic training-images (I_{S}).

6. The training-device (1) according to one of the preceding claims, wherein the testing-unit (3) is designed for:
- selecting testing-images (T) with test qualities below a predefined quality-threshold,
- evaluating patterns in the second sets of parameter-values (P2) of these testing-images (T),
- determining parameter value-ranges (V) from these patterns,
- forming a first set of parameter-values (P1) based on the determined value-ranges (V).

7. A method for training a machine learning model (M) with a training-device (1) according to one of the preceding claims, comprising the steps:
a) providing training-images (I) and testing-images (T),
b) training of the model (M) with the training-images (I),
c) testing the trained model (M) based on the testing-images (T), wherein individual test-qualities are evaluated for single testing-images (T), and parameter value-ranges (V) are determined for groups of testing-images (T), representing value-ranges (V) with a relatively poor image-density, where test-qualities are below a predefined threshold value, and/or the relative number of training-images (I) compared to other parameter value-ranges (V) is below a predefined threshold,
d) generating synthetic training-images (I_{S}) and preferably also synthetic testing-images (T_{S}) with the data-generator (4) based on the evaluated set of parameter value-ranges (V),
e) repeating at least steps b) and c) with the generated synthetic training-images (I_{S}) and preferably also with generated synthetic testing-images (T_{S}).

8. The method according to claim 7, wherein for step a) at least a part of the training-images (I) and/or the testing-images are synthetic images generated by the data-generator (4).

9. The method according to one of claims 7 to 8, wherein the image-density of parameter value-ranges (V) are evaluated by evaluating the performance of the testing with testing-images (T), and when the trained model (M) has poor performance on specific cases, respective parameter value-ranges (V) are determined.

10. The method according to one of claims 7 to 9, wherein the image-density of parameter value-ranges (V) are evaluated by counting the images in each range and normalizing the counted values,
preferably wherein the image-density of parameter value-ranges (V) are evaluated by counting the images with a specific test result in each range and normalizing the counted values.

11. The method according to one of claims 7 to 10, wherein parameter value-ranges (V) are defined by prior knowledge about a relatively poor image-density in these value-ranges (V) and synthetic training-images (I_{S}) are generated by the data-generator (4), and preferably also synthetic testing-images (T_{S}) for testing.

12. The method according to one of claims 7 to 11, wherein multiple training-iterations are performed with synthetic training-images (I_{S}), and preferably also synthetic testing-images (T_{S}), based on certain sets of parameter-values (P1), preferably wherein during each training iteration sets of parameter-values (P1) are evaluated from the testing-images (T) and if testing-images (T) of a set of parameter-values (P2) perform poorly, this set of parameter-values (P2) is prioritized as new first set of parameter-values (P1) in the next iteration, prompting the generator to produce more data with this first set of parameter-values (P1).

13. The method according to one of claims 7 to 12, wherein the training is based on supervised learning, while each training-image and testing-image is connected with or comprises an individual ground truth, preferably wherein the training is performed in order to segment the images and/or classify elements of the images and/or detect objects in the images.

14. A computer program product comprising instructions which, when the program is executed by a computer, cause the computer to carry out the steps of the method of any of claims 7 to 13.

15. A computer-readable storage medium comprising instructions which, when executed by a computer, cause the computer to carry out the steps of the method of any of claims 7 to 13.
